# EUROPEAN PATENT SPECIFICATION

(11) **EP 3 845 216 B1**
(45) Date of publication and mention of the grant of the patent: **09.04.2025**
(21) Application number: 19854281.3
(22) Date of filing: 29.08.2019
(51) Int. Cl.: A61K 9/00, A61K 47/38, A61K 47/34

(54) **IMPLANTABLE SUSTAINED-RELEASE MICRONEEDLE PATCH AND PREPARATION METHOD THEREFOR**
IMPLANTIERBARES MIKRONADEL-PFLASTER MIT VERZÖGERTER FREISETZUNG UND VERFAHREN ZU SEINER HERSTELLUNG
PATCH À MICRO-AIGUILLE IMPLANTABLE À LIBÉRATION PROLONGÉE ET PROCÉDÉ DE PRÉPARATION ASSOCIÉ

(30) Priority: 31.08.2018 CN 201811011984
(43) Date of publication of application: 07.07.2021
(73) Proprietor: Beijing Cas Microneedle Technology Ltd., Beijing 102600 (CN)
(72) Inventor: GAO, Yunhua, Beijing 102600 (CN); HE, Meilin, Beijing 102600 (CN); YANG, Guozhong, Beijing 102600 (CN); ZHANG, Suohui, Beijing 102600 (CN)
(74) Representative: Wachinger, Julian Friedrich
(86) International application number: PCT/CN2019/103332
(87) International publication number: WO 2020/043168

(56) References cited:
- CN-A- 107 405 301
- CN-A- 107 811 963
- CN-A- 108 325 064
- CN-A- 108 392 729
- US-A1- 2011 046 575
- US-A1- 2014 276 589

## Description

### TECHNICAL FIELD

The present invention relates to the field of medical technology. More specifically, it relates to an implantable sustained-release microneedle patch and a preparation method therefor.

### BACKGROUND

Transdermal drug delivery preparations are a dosage form by which drugs are administered through skin, which can avoid the interference of the gastrointestinal environment with the drug effect and the "first pass effect" of the liver, maintain a constant optimal plasma-drug concentration or physiological effect, prolong the effective acting time, and reduce the dosage frequency. The drugs can be administrated by patients on their own, and the patient compliance is good. However, the stratum corneum in the outer layer of the skin will hinder the absorption of drugs, and the drugs are not easy to penetrate into the body, resulting in very limited choices of drugs. In recent years, microneedle technology has received widespread attention. It is one of physical penetration enhancement methods using transdermal drug delivery, which can achieve painless and precise drug delivery.

Traditional microneedles made of metal, glass, and silicon materials will inevitably be broken and left in the skin during use due to the properties of their materials, causing damage to the human body. In recent years, the emerging polymer microneedles use materials including water-soluble polymer that can be absorbed by the skin, biocompatible polymer, and biodegradable polymer materials, greatly reducing the risk of use; furthermore, polymer microneedles are advantageous in low production cost, simple manufacturing process, mass production, and environmental friendliness, and microneedle manufacturing can achieve controlled release of drugs by selecting water-soluble polymer materials or biodegradable polymer materials having different physical and chemical properties. In recent years, many scientific researchers have devoted themselves to using polylactic acid-based degradable polymer materials to make polymer microneedles that are biocompatible, naturally degradable, and easy to prepare.

Chinese patent (CN104888343 A) discloses a polymer solid microneedle and a batched preparation method therefor. The material described in this invention is a biodegradable water-insoluble polymer material. The preparation method used is to place polymer material particles on a microneedle mold, heat the particles in a closed heating device until the particles melt, and use the preparation device to implement press molding of the melt while the melt is hot. However, the microneedle prepared in this patent cannot load drugs, and the microneedle only plays a role in pretreating the skin and destroying the skin stratum corneum barrier during use.

International patent (WO2007/030477) discloses a solid drug solution perforator containing drug particles and/or drug-adsorbed or loaded particles with an associated drug reservoir (SSPP system). In order to deliver drugs, the SSPP system comprises an active drug ingredient in a particulate form or a drug adsorbed on a particle surface in a matrix material that dissolves upon contact with a patient's body. The inert particles are poly(lactic-co-glycolic acid) (PLGA) or aluminum hydroxide and aluminum phosphate. The microneedle made by this method can be loaded with protein and vaccine drugs. However, in this method, the drug barely has a sustained-release effect since it is adsorbed on the PLGA inert particles.

Literature 1 (Pharmaceutical Research. 2006 May;23(5):1008-19.) proposes a method for preparing a microneedle by using poly(lactic-co-glycolic acid) (PLGA) as a material of a microneedle support. The method achieves controlled release of drugs by loading into the microneedle the drugs or microspheres of polylactic acid or sodium carboxymethyl cellulose loaded with the drugs. However, in this method, since the matrix material of the body of the microneedle is PLGA, the user needs to apply it for a long time until the material PLGA of the support is completely degraded.

Literature 2 (Biomed Microdevices. 2007 Apr;9(2):223-34) proposes a method for preparing a porous multi-layered biodegradable microneedle by using PLA, PGA or PLGA microspheres. The method is to prepare a porous or multi-layered microneedle by injecting microspheres made of a biodegradable polymer material into a mold, and using extrusion and ultrasonic welding or thermal welding technology. In the microneedle manufacturing, the microspheres need to be prepared by spray drying technology or by emulsification at first, and thus the process is complicated. Compared with a single microneedle, the porous multi-layered microneedle is weaker and easier to break, and is difficult to be effectively demolded from the mold. US2014/2765889 1 discloses an implantable sustained-release microneedle, comprising a needle tip, a needle body, and a base; the needle tip comprising a needle tip center layer and a needle tip outer layer; the needle tip center layer being formed of a matrix comprising a biodegradable water-insoluble polymer material; and the needle tip outer layer in form of an adhesive, the needle body and the base being formed of a matrix comprising a water-soluble polymer material. US 2011/046575 discloses the use of water-soluble polymer material in such microneedle (example 24 chondroitin sulfate C).

In summary, from the current point of view, taking polylactic acid microneedles as an example, the manufacturing of microneedles can be divided into two categories: one is to first make polylactic acid-based biodegradable polymer materials and drugs into microspheres, then add them into a microneedle mold, and then heat, melt, and cool them to form a microneedle with a certain mechanical strength, such microneedle can be loaded with drugs, but it needs to make microspheres at first to form the microneedle, the operation is cumbersome and the cost is high; the other is to directly add polylactic acid powder into a microneedle mold, and then heat, melt (above 200 °C) and then cool them to form a microneedle, but this manufacturing method is difficult to load drugs.

Therefore, there is a need to provide a polylactic acid-based implantable sustained-release microneedle which is simple in process and high in safety, and does not need long-term application.

### SUMMARY

A first objective of the present invention is to provide an implantable sustained-release microneedle, which has sufficient mechanical strength and can implant a polylactic acid-based needle tip into the skin, realizing purposes of convenience, safety, biodegradability, and efficient administration.

The second objective of the present invention is to provide a preparation method for the above-mentioned implantable sustained-release microneedle, which has simple process and low cost.

The third objective of the present invention is to provide a microneedle patch comprising the above-mentioned implantable sustained-release microneedle.

To achieve the above objectives, the present invention adopts the following technical solutions:
The present invention provides an implantable sustained-release microneedle, comprising a needle tip, a needle body, and a base; the needle tip comprises a needle tip center layer and a needle tip outer layer; the needle tip center layer is formed of a matrix comprising a biodegradable water-insoluble polymer material; and the needle tip outer layer, the needle body, and the base are formed of a matrix comprising a water-soluble polymer material.

Further, the biodegradable water-insoluble polymer material includes, but is not limited to, one or more of polylactic acid, poly-L-lactide, poly-DL-lactide, poly(lactic-co-glycolic acid), polyglycolic acid, and polycaprolactone.

In the present invention, the water-soluble polymer material of the needle tip outer layer of the microneedle is connected with the water-soluble polymer material of the needle body of the microneedle, which can enhance the mechanical strength of the microneedle and improve the success rate of the microneedle puncturing the skin. Without the wrapping by the outer layer of the microneedle, since the biodegradable material of the center layer of the microneedle has weak hydrophilicity, and the strength of connection with the water-soluble polymer matrix material of the needle body is low, the microneedle will easily break at the time of puncturing, the success rate of the needle tip entering the skin is relatively low, and the quality of the microneedle puncturing the skin is uncontrollable.

Further, the needle tip center layer comprises at least one active ingredient; preferably, a mass ratio of the biodegradable water-insoluble polymer material to the active ingredient is 0.5:1-1000:1, so as to ensure the mechanical strength and skin penetration of the microneedle.

In a preferred embodiment of the present invention, the needle tip center layer further comprises a pore-forming agent. The pore-forming agent helps intradermal water molecules enter the interior of the matrix of the needle tip center layer, so as to regulate the drug release rate. The pore-forming agent includes, but is not limited to, one or more of sodium chloride, sodium carbonate, sodium bicarbonate, ammonium bicarbonate, trehalose, maltose, polyethyleneglycol, cyclodextrin and its derivatives, polyvinylpyrrolidone (PVP), a low-molecular weight hyaluronic acid and its sodium salt (with a molecular weight of 5-100 kDa), and low-molecular weight cellulose derivatives (with a molecular weight of 5-100 kDa).

Preferably, the pore-forming agent accounts for 0.1%-10% of the total mass of the needle tip center layer.

In a preferred embodiment of the present invention, the needle tip center layer further comprises a protective agent. The protective agent includes, but is not limited to, one or more of polyhydroxy compounds (mannitol, sorbitol, xylitol, polyethyleneglycol, etc.), carbohydrate compounds (trehalose, dextrin, lactose, sucrose, maltose, etc.), serum albumin, polyvinylpyrrolidone, chondroitin sulfate, amino acids (proline, tryptophan, glutamic acid, glycine, etc.)

Preferably, the protective agent accounts for 0.1%-10% of the total mass of the needle tip center layer.

Further, the water-soluble polymer material includes, but is not limited to, one or more of carboxymethyl cellulose, hydroxypropyl methyl cellulose (HPMC), hydroxyethyl cellulose, polyvinyl alcohol and its derivatives, polyvinylpyrrolidone (PVP) and its derivatives, sodium hyaluronate and its derivatives, chondroitin sulfate, chitosan derivatives, polyacrylamide derivatives, polyglutamic acid, polydopamine, pullulan, gelatin, collagen, plant protein, and silk protein.

Preferably, the molecular weight of the water-soluble polymer material is 20-2000 kDa.

Preferably, the needle tip outer layer, the needle body, and the base further comprise low-molecular weight carbohydrates and polyol compounds (specifically, one or more of trehalose, sucrose, maltose, sorbitol, mannitol, xylitol, and glycerol, etc.), so as to accelerate a dissolution rate of the water-soluble polymer material.

Preferably, the needle tip outer layer, the needle body, and the base are the same type of water-soluble polymer material.

Further, the needle tip has a conical shape or a polygonal conical shape, preferably, the needle tip has a conical shape; the density of the needle tip of the microneedle is: 25-1000 needle bodies and needle tips per square centimeter of the base; the height of the needle tip and the needle body is 0.3-2 mm; the angle of the needle tip is 10-60°; and the thickness of the base is 10-300 µm. The height of the needle tip center layer is not greater than two-thirds of the height of the needle tip and the needle body.

The present invention further provides a preparation method for the above-mentioned implantable sustained-release microneedle, comprising the following steps:
1) mixing a biodegradable water-insoluble polymer material with a part of an organic solvent, adding or not adding a pore-forming agent, adding or not adding a protective agent, to prepare a needle tip center layer matrix material solution; mixing an active ingredient with the remaining organic solvent to prepare a drug solution; mixing the drug solution with the needle tip center layer matrix material solution to obtain a needle tip center layer injection molding liquid;
   alternatively,
   mixing the biodegradable water-insoluble polymer material with the organic solvent, adding or not adding the pore-forming agent, adding or not adding the protective agent, adding the active ingredient, and mixing uniformly to obtain the needle tip center layer injection molding liquid;
2) mixing the water-soluble polymer material with water, adding or not adding low-molecular weight carbohydrates or polyol compounds, and mixing uniformly to obtain a needle tip outer layer injection molding liquid; mixing the water-soluble polymer material with water, adding or not adding the low-molecular weight carbohydrates or the polyol compounds, and mixing uniformly to obtain a needle body and base injection molding liquid;
3) injecting the needle tip outer layer injection molding liquid into a microneedle mold, and drying to prepare a needle tip outer layer; injecting the needle tip center layer injection molding liquid into the microneedle mold, heating to remove the organic solvent, and cooling; injecting the needle body and base injection molding liquid into the microneedle mold to prepare a needle body and a base, drying, and demolding to obtain the implantable sustained-release microneedle.

Further, the organic solvent includes, but is not limited to, acetone, ethylacetate, chloroform, dichloro or N-methylpyrrolidone; preferably, the organic solvent is N-methylpyrrolidone.

Further, the mass concentration of the biodegradable water-insoluble polymer material in the needle tip center layer injection molding liquid is 5-30%; the mass concentration of the water-soluble polymer material in the needle tip outer layer injection molding liquid is 0.5-10%; the mass concentration of the water-soluble polymer material in the needle body and base injection molding liquid is 10-50%; and the mass ratio of the biodegradable water-insoluble polymer material to the active ingredient is 0.5:1-1000:1.

Further, in said step 3), the needle tip center layer injection molding liquid, the needle tip outer layer injection molding liquid, or the needle body and base injection molding liquid are added to the microneedle mold by means of a pressurization method or a vacuum method, so as to avoid the formation of air bubbles in the needle during manufacturing. If the pressurization method is adopted, then the applied pressure is 0.2-0.6 MPa, and the pressurization time is 1-20 min. If the vacuum method is adopted, then the vacuum degree is 0.05-0.1 MPa, and the vacuuming time is 3-20 min.

Preferably, the drying time after the needle tip outer layer injection molding liquid is injected into the microneedle mold is 1-10 min.

Preferably, the heating temperature after the needle tip center layer injection molding liquid is injected into the microneedle mold is 30-85 °C, and the heating time is 1-24 h, so as to volatilize the organic solvent and ensure that the microneedle has sufficient mechanical strength and skin puncturing ability after cooling.

Preferably, the drying condition after the needle body and base injection molding liquid is injected into the microneedle mold is drying for 0.5-6 hours under 25-30 °C and 10-35% humidity.

The present invention further provides an implantable sustained-release microneedle patch comprising the above-mentioned implantable sustained-release microneedle and a backing; preferably, the backing is a pressure-sensitive adhesive backing or a silicone backing or a hydrocolloid backing.

The preparation method for the above-mentioned implantable sustained-release microneedle patch is to, based on preparing the implantable sustained-release microneedle, further paste the backing on the backside of the dried base and then demould to obtain the implantable sustained-release microneedle patch.

The implantable sustained-release microneedle patch described in the present invention can be applied in fields of disease treatment and prevention, health care, and cosmetology.

The term "active ingredient" refers to a substance used for diagnosis, treatment, prevention, cosmetic or health care purposes that is delivered through the microneedle or the microneedle patch of the present invention in a transdermal manner and has the efficacy of acting on animals or humans. According to the present invention, the active ingredient includes, but is not limited to, pharmaceutical active ingredients, vaccine active ingredients, cosmetic active ingredients, health care product active ingredients, etc., which are specifically selected according to actual needs.

### The beneficial effects of the present invention are as follows:

In the microneedle or the microneedle patch of the present invention, a biodegradable water-insoluble polymer material is used to make the needle tip center layer of the microneedle, and a water-soluble polymer material is used to make the needle body, the base, and the needle tip outer layer, so as to form a microneedle having a needle tip center layer insoluble in water. After acting on the skin, the water-soluble material of the needle tip outer layer and the needle body absorbs the moisture within the skin, so that the needle body and the base are quickly separated from the needle tip within 0.5-1 h; and after the base of the patch is removed, the needle tip of the microneedle will be completely left in skin, and thus weeks of long-term in vivo release of a drug can be ensured without a user sticking the microneedle patch for a long time. Such microneedle patches are not limited by the manufacturing area, and the drug load can be greatly increased by extending the area, which is suitable for long-term intradermal release of various drugs. In addition, the preparation process for the microneedle or the microneedle patch of the present invention avoids the cumbersome process such as encapsulating the drug in the microsphere liposome, but uses a low-toxic organic solvent such as N-methylpyrrolidone to dissolve biodegradable polylactic acid-based polymer materials, the process cost is reduced, and the process is similar to conventional manufacturing methods for a dissolvable microneedle or a microneedle patch, the operation is simple and fast, high temperature melting is not required, and the applicable drug range is wide.

### DESCRIPTION OF THE DRAWINGS

The specific embodiments of the present invention will be described in further details below with reference to the accompanying drawings.
FIG. 1 shows a schematic structural view of a microneedle patch.
FIG. 2 shows a view of dissolution of a microneedle patch: a needle tip is completely dissolved in the skin in 60 minutes.
FIG. 3 shows a curve of in vitro transdermal release of a microneedle patch, and an inserted graph shows a curve of a cumulative release amount in the first 60 h.
FIG. 4 shows a photograph of a microneedle patch under a stereo microscope.
FIG. 5 shows a side view of a microneedle patch under a stereo microscope.
FIG. 6 shows a skin puncturing ability of a microneedle patch.
FIG. 7 shows a photograph of a polylactic acid part of a needle tip of a microneedle patch implanted in pigskin under a fluorescence microscope.

### DETAILED DESCRIPTION

In order to explain the present invention more clearly, the present invention will be further described below in conjunction with preferred examples and accompanying drawings. Similar components in the accompanying drawings are denoted by the same reference numerals. Those skilled in the art should understand that the content described in detail below is illustrative rather than restrictive, and should not limit the protection scope of the present invention.

### Example 1 Preparation of a polylactic acid-based implantable sustained-release microneedle patch

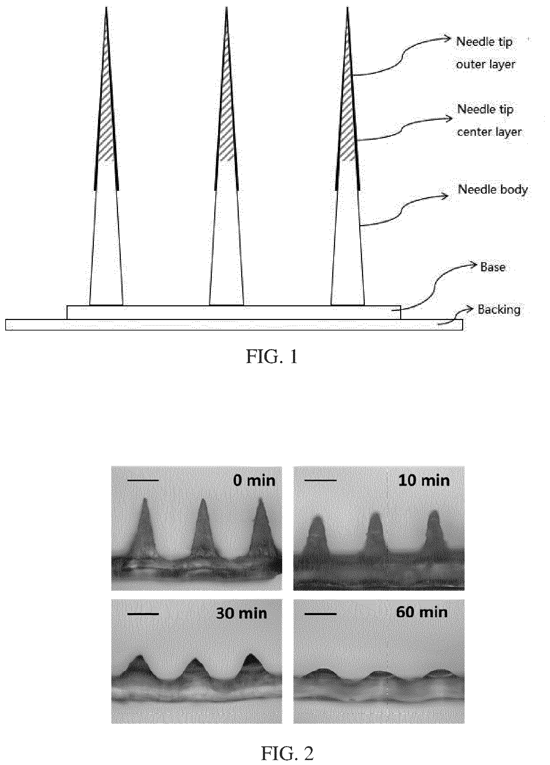

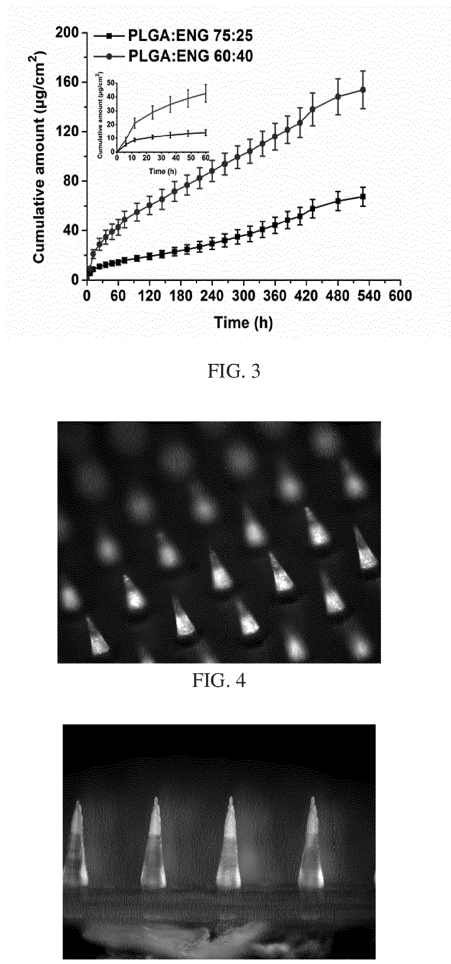

1. 0.05g of polyvinyl alcohol with a molecular weight of 200 kDa was taken and weighed, 4.95 ml of water was added, the resulting solution was placed and heated under 85 °C for 1 hour, a polyvinyl alcohol aqueous solution with a mass fraction of 1% was prepared as a needle tip outer layer injection molding liquid; and 3.5 g of polyvinyl alcohol with a molecular weight of 20 kDa was taken and weighed. 6.5 ml of water was added, the resulting solution was placed and heated under 85 °C for 2 hours, a polyvinyl alcohol aqueous solution with a mass fraction of 30% was prepared as a needle body and base injection molding liquid.
2. 0.3 g of PLGA (80/20) with a molecular weight of 20 kDa was taken and weighed, and 0.7 ml of N-methylpyrrolidone was added, to prepare a PLGA solution with a mass fraction of 30%; 50 mg of etonogestrel (ENG) was taken and weighed, and 1 ml of N-methylpyrrolidone was added, to prepare a 50 mg/ml etonogestrel solution; the above-mentioned etonogestrel solution and the above-mentioned PLGA solution were mixed uniformly in a certain ratio to prepare solutions containing PLGA: ENG at solid content ratios of 60: 40 and 75: 25, respectively, as the needle tip center layer injection molding liquid.
3. 10 µl of the above-mentioned needle tip outer layer injection molding liquid was added into a mold, the needle tip outer layer injection molding liquid was forced into pinholes of the microneedle mold by means of vacuuming, the excess solution on the mold was removed, and the mold was placed under 25 °C and 20% humidity to dry for 10 minutes; the above-mentioned needle tip center layer injection molding liquid was added into the above-mentioned microneedle mold, the needle tip center layer injection molding liquid was forced into the pinholes of the above-mentioned microneedle mold by means of vacuuming, the excess solution on the mold was removed, and the mold was heated under 80 °C for 2 hours; 150 µl of the above-mentioned base injection molding liquid was added to the above-mentioned cooled microneedle mold and vacuumed to avoid the formation of air bubbles; the above-mentioned microneedle mold was placed under 25 °C and 10% humidity to dry for 6 hours.
4. A pressure-sensitive adhesive backing was pasted on a backside of the base of the dried microneedle, and the microneedle patch was demoulded.
   The prepared microneedle patch is as shown in FIG. 1. There are 400 needles in per square centimeter of the microneedle patch, and the height of the needle tip and the needle body is 0.5 mm.
5. The microneedle patch was applied to a surface of pigskin, the patch was peeled off at different time points, and a dissolution situation of the needle tip of the microneedle patch was observed under a microscope. As shown in FIG. 2, it can be seen from the figure that the needle tip was completely dissolved in skin in 60 minutes.
6. In vitro transdermal experiment: the microneedle patch was applied to the surface of pigskin, as for a transdermal cup, a medium of a receiving solution in the cup was a 30% PEG400-10% ethanol-PBS buffer solution, samples were taken at a predetermined time point in a full sampling manner, and an equal volume of the receiving medium was complemented immediately after each sampling. FIG. 3 is a curve of in vitro transdermal release, showing that the microneedle patch prepared in Example 1 has a good sustained-release effect, and the sustained-release time can reach 5-6 weeks.

In this example, etonogestrel can be replaced by other active ingredients of small molecular compounds having thermal stability at 80 °C, which include, but are not limited to ethinyl estradiol, levonorgestrel, norgestrel, gestodene, desogonolone, desogestrel, artemisinin derivatives, paclitaxel derivatives and other active ingredients, and the obtained microneedle patch has a similar sustained-release effect.

The polyvinyl alcohol in the needle tip outer layer injection molding liquid and the needle body and base injection molding liquid of the this example can be replaced by carboxymethyl cellulose, hydroxypropyl methyl cellulose, hydroxyethyl cellulose, polyvinyl alcohol derivatives, polyvinylpyrrolidone and its derivatives, sodium hyaluronate and its derivatives, chondroitin sulfate, chitosan derivatives, polyacrylamide derivatives, polyglutamic acid, polydopamine, pullulan, gelatin, collagen, and silk protein, and the obtained microneedle patch has a similar sustained-release effect.

In the needle tip center layer injection molding liquid of this example, a pore-forming agent such as 0.1-5% sodium chloride, 0.1-5% sodium carbonate, 0.1-3% sodium bicarbonate, 0.1-3% ammonium bicarbonate, 1-10% trehalose, 1-10% maltose, 1-10% cyclodextrin and its derivatives, 1-5% polyvinylpyrrolidone, 1-5% sodium hyaluronate, and 1-5% carboxymethyl cellulose can be further added, and the obtained microneedle patch can be sustained-released for 5-20 days.

### Example 2 Preparation of a polylactic acid-based implantable sustained-release microneedle patch

1. 0.05 g of sodium carboxymethyl cellulose with a molecular weight of 200 kDa was taken and weighed, and 9.95 ml of water was added, to prepare a sodium carboxymethyl cellulose aqueous solution with a mass fraction of 0.5% as the needle tip outer layer injection molding liquid; 2.5 g of sodium carboxymethyl cellulose with a molecular weight of 200 kDa was taken and weighed, and 7.5 ml of water was added, to prepare a sodium carboxymethyl cellulose aqueous solution with a mass fraction of 25% as the needle body and base injection molding liquid.
2. 0.2 g of polylactic acid (PLA) with a molecular weight of 10 kDa was taken and weighed, and 0.6 ml of N-methylpyrrolidone was added, to prepare a PLA solution with a mass fraction of 20%; 10 mg of a fat-soluble simulated drug red fluorescent dye was taken and weighed, and 1 ml of N-methylpyrrolidone was added, to prepare a red fluorescent dye solution with a mass fraction of 10 mg/ml; 0.5 ml of the red fluorescent dye solution was mixed uniformly with 0.5 ml of the PLA solution, to prepare a 10% PLA solution containing 5 mg/ml of the red fluorescent dye as the needle tip center layer injection molding liquid.
3. 20 µl of the needle tip outer layer injection molding liquid was added into a mold, the needle tip outer layer injection molding liquid was forced into pinholes of the microneedle mold by means of pressurization, the excess solution on the mold was removed, and the mold was placed under 25 °C and 30% humidity to dry for 10 minutes; the above-mentioned needle tip center layer injection molding liquid was added into the above-mentioned microneedle mold, the needle tip center layer injection molding liquid was forced into the pinholes of the above-mentioned microneedle mold by means of vacuuming, the excess solution on the mold was removed, and the mold was heated under 80 °C for 2 hours; 150 µl of the above-mentioned needle body and base injection molding liquid was added to the above-mentioned cooled microneedle mold and vacuumed to avoid the formation of air bubbles; the above-mentioned microneedle mold was placed under 25 °C and 30% humidity to dry for 0.5 hours.
4. A pressure-sensitive adhesive backing was pasted on a backside of the base of the dried microneedle, and the microneedle patch was demoulded.

The prepared microneedle patch is as shown in FIG. 1. Photographs taken by a stereo microscope are as shown in FIGS. 4-5. There are 400 needles in per square centimeter of the microneedle patch, and the height of the needle tip and the needle body is 0.6 mm.

If the microneedle is applied to pigskin and peeled off from the base within 30 minutes, it can be sustained-released for one month.

In this example, the red fluorescent dye can be replaced by other active ingredients of small molecular compounds with a heat resistance of 80 °C, and the obtained microneedle patch has a similar sustained-release effect.

### Example 3 Preparation of a polylactic acid-based implantable sustained-release microneedle patch

1. 0.25 g of carboxymethyl cellulose with a molecular weight of 40 kDa was taken and weighed, and 4.75 ml of water was added, to prepare a carboxymethyl cellulose aqueous solution with a mass fraction of 5% as the needle tip outer layer injection molding liquid; 3 g of polyvinyl alcohol with a molecular weight of 70 kDa was taken and weighed, 7ml of water was added, and the resulting solution was heated under 85 °C for 2 hours, to prepare a polyvinyl alcohol aqueous solution with a mass fraction of 30% as a needle body and base injection molding liquid.
2. 0.4 g of PLGA (75/25) with a molecular weight of 10 kDa was taken and weighed, and 0.6 ml of N-methylpyrrolidone was added, to prepare a PLGA solution with a mass fraction of 30%; 5 mg of interferon a-2b was taken and weighed and dissolved in 1 ml of sterile water for injection, 10 mg of zinc hydroxide subjected to micronization and sterilization was added, the resulting solution was vortex-mixed for 10 minutes to form a zinc salt-containing interferon a-2b solution; the zinc salt-containing interferon a-2b solution was added to the above-mentioned 30% PLGA solution and stirred to form a drug-loaded sol system as the needle tip center layer injection molding liquid.
3. 20 µl of the above-mentioned needle tip outer layer injection molding liquid was added into a mold, the needle tip outer layer injection molding liquid was forced into pinholes of the microneedle mold by means of pressurization, the excess solution on the mold was removed, and the mold was placed under 25 °C and 30% humidity to dry for 10 minutes; the above-mentioned needle tip center layer injection molding liquid was added into the above-mentioned microneedle mold, the needle tip center layer injection molding liquid was forced into the pinholes of the above-mentioned microneedle mold by means of vacuuming, the excess solution on the mold was removed, and the mold was heated under 30 °C for 24 hours; 200 µl of the above-mentioned needle body and base injection molding liquid was added to the above-mentioned cooled microneedle mold and vacuumed to avoid the formation of air bubbles; the above-mentioned microneedle mold was placed under 25 °C and 10% humidity to dry for 6 hours.
4. A pressure-sensitive adhesive backing was pasted on a backside of the base of the dried microneedle, and the microneedle patch was demoulded.

The prepared microneedle patch is as shown in FIG. 1. There are 1000 needles in per square centimeter of the microneedle patch, and the height of the needle tip and the needle body is 0.3 mm.

If the microneedle patch is applied to pigskin, the base can be completely peeled off within 1 hour, and the microneedle patch can be sustained-released for 4 weeks according to in vitro transdermal results.

In this example, the interferon a-2b can be replaced by other types of interferons, and can also be replaced by other proteins and polypeptides or hydrophilic small molecules, such as insulin, growth hormones, cytokines, nerve growth factors, amino acids, etc. The obtained needle patch has a similar sustained-release effect.

The carboxymethyl cellulose in the needle tip outer layer injection molding liquid and the polyvinyl alcohol in the needle body and base injection molding liquid of the this example can be replaced by carboxymethyl cellulose, hydroxypropyl methyl cellulose, hydroxyethyl cellulose, polyvinyl alcohol derivatives, polyvinylpyrrolidone and its derivatives, sodium hyaluronate and its derivatives, chondroitin sulfate, chitosan derivatives, polyacrylamide derivatives, polyglutamic acid, polydopamine, pullulan, gelatin, collagen, and silk protein, and the obtained microneedle patch has a similar sustained-release effect.

In the needle tip center layer injection molding liquid of this example, a pore-forming agent such as 0.1-5% sodium chloride, 0.1-5% sodium carbonate, 0.1-3% sodium bicarbonate, 0.1-3% ammonium bicarbonate, 1-10% trehalose, 1-10% maltose, 1-10% cyclodextrin and its derivatives, 1-5% polyvinylpyrrolidone, 1-5% sodium hyaluronate, and 1-5% carboxymethyl cellulose can be added, and the sustained-release time of the obtained microneedle patch ranges between 5-20 days.

A protective agent can be added to the needle tip center layer injection molding liquid of this example, such as one or more of 1-10% polyhydroxy compounds (such as 1% glycerin, 3% butylene glycol, 1-5% xylitol, 1-10% mannitol), 1-10% carbohydrate compounds (1-10% trehalose, 1-5% sucrose), 0.1-5% serum albumin, 1-10% polyvinylpyrrolidone, and 1-10% amino acids, and the sustained-release time of the obtained microneedle patch ranges between 5-20 days.

In the carboxymethyl cellulose in the needle tip outer layer injection molding liquid and the needle body and base injection molding liquid of this example, low-molecular weight carbohydrates and polyol compounds, specifically 1-10% trehalose , 1-5% sucrose, 1-10% sorbitol, 5-10% mannitol, 5-10% xylitol, 0.1-3% glycerol or the like can further be added to accelerate a dissolution rate of the water-soluble polymer materials, and the sustained-release effect of the obtained microneedle patch is similar to this example.

### Example 4 Preparation of a polylactic acid-based implantable sustained-release microneedle patch

1. 1g of chondroitin sulfate with a molecular weight of 40 kDa is taken and weighed, and 9 ml of water was added, to prepare a chondroitin sulfate aqueous solution with a mass fraction of 10% as the needle tip outer layer injection molding liquid; 4 g of chondroitin sulfate with a molecular weight of 40 kDa was taken and weighed, and 6 ml of water was added, to prepare a chondroitin sulfate aqueous solution with a mass fraction of 40% as the needle body and base injection molding liquid.
2. N-methylpyrrolidone was used as a solvent to prepare solutions containing 5%, 15%, and 30% PLGA, respectively, and 10 mg of granisetron hydrochloride was added to the above-mentioned solutions to prepare needle tip center layer injection molding liquids containing PLGA: granisetron hydrochloride in mass ratios of 1:2, 1.5:1, and 3:1.
3. 20 µl of the above-mentioned needle tip outer layer injection molding liquid was added into a mold, the needle tip outer layer injection molding liquid was forced into pinholes of the microneedle mold by means of pressurization, the excess solution on the mold was removed, and the mold was placed under 25 °C and 30% humidity to dry for 15 minutes; the above-mentioned needle tip center layer injection molding liquid was added into the above-mentioned microneedle mold, the needle tip center layer injection molding liquid was forced into the pinholes of the above-mentioned microneedle mold by means of vacuuming, the excess solution on the mold was removed, and the mold was heated under 60 °C for 4 hours; 150 µl of the above-mentioned needle body and base injection molding liquid was added to the above-mentioned cooled microneedle mold and vacuumed to avoid the formation of air bubbles; the above-mentioned microneedle mold was placed under 25 °C and 35% humidity to dry for 1 hour.
4. A pressure-sensitive adhesive backing was pasted on a backside of the base of the dried microneedle, and the microneedle patch was demoulded.

The prepared microneedle patch is as shown in FIG. 1. There are 169 needles in per square centimeter of the microneedle patch, and the height of the needle tip and the needle body is 0.8 mm.

If the microneedle patch is applied to pigskin, it can be separated from the needle tip after 30 minutes, and the sustained-release time of the drug is respectively 14 days, 18 days, and 21 days, according to in vitro transdermal results. As the content of PLGA increases, the sustained-release time extends.

In this example, granisetron hydrochloride can also be replaced by leuprolide acetate, octreotide acetate, amlodipine besylate/ketoprofen, cyclosporine, diclofenac sodium controlled release, everolimus, methylphenidate, clarithromycin, mycophenolic acid, griseofulvin, mabilone, tacrolimus and other drugs, and the obtained microneedle patch has a similar sustained-release effect.

### Example 5 Preparation of a polylactic acid-based implantable sustained-release microneedle patch in which a pore-forming agent is added in a needle tip center layer

1. 0.05 g of polyvinyl alcohol with a molecular weight of 50 kDa was taken and weighed, 4.95 ml of water was added, the resulting solution was placed and heated under 85 °C for 1 hour, a polyvinyl alcohol aqueous solution with a mass fraction of 1% was prepared as a needle tip outer layer injection molding liquid; and 3.5 g of polyvinyl alcohol with a molecular weight of 50 kDa was taken and weighed. 6.5 ml of water was added, the resulting solution was placed and heated under 85 °C for 2 hours, a polyvinyl alcohol aqueous solution with a mass fraction of 35% was prepared as a needle body and base injection molding liquid.
2. 3 g of PLGA (80/20) with a molecular weight of 20 kDa was taken and weighed, 7 ml of N-methylpyrrolidone was added, and then 0.03 g of PVP K30 was added, to prepare a PLGA solution containing PVP K30; 50 mg of vinpocetine was taken and weighed, and 1 ml of N-methylpyrrolidone was added, to prepare a 50 mg/ml vinpocetine solution; 1 ml of the vinpocetine solution was mixed with 1 ml of the PLGA solution containing PVP K30 to prepare a needle tip center layer injection molding solution containing vinpocetine.
3. 10 µl of the above-mentioned needle tip outer layer injection molding liquid was added into a mold, the needle tip outer layer injection molding liquid was forced into pinholes of the microneedle mold by means of vacuuming, the excess solution on the mold was removed, and the mold was placed under 25 °C and 20% humidity to dry for 10 minutes; the above-mentioned needle tip center layer injection molding liquid was added into the above-mentioned microneedle mold, the needle tip center layer injection molding liquid was forced into the pinholes of the above-mentioned microneedle mold by means of vacuuming, the excess solution on the mold was removed, and the mold was heated under 70 °C for 3 hours; 150 µl of the above-mentioned needle body and base injection molding liquid was added to the above-mentioned cooled microneedle mold and vacuumed to avoid the formation of air bubbles; the above-mentioned microneedle mold was placed under 25 °C and 10% humidity to dry for 6 hours.
4. A pressure-sensitive adhesive backing was pasted on a backside of the base of the dried microneedle, and the microneedle patch was demoulded.

The prepared microneedle patch is as shown in FIG. 1. There are 25 needles in per square centimeter of the microneedle patch, and the height of the needle tip and the needle body is 2 mm.

If the microneedle patch is applied to pigskin, the base can be completely peeled off within 30 minutes, and the microneedle patch can be sustained-released for 3 weeks.

### Examples 6-17

Polylactic acid-based sustained-release microneedle patches comprising a pore-forming agent were prepared respectively according to the preparation method of Example 5. The specific formulations are shown in Table 1.

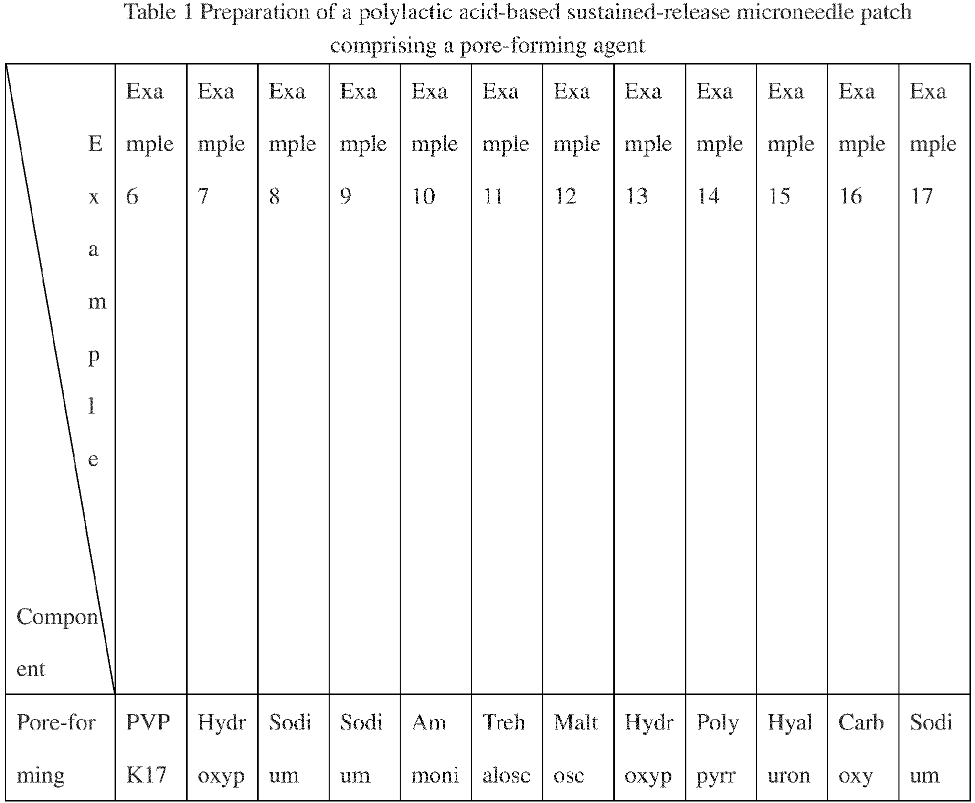

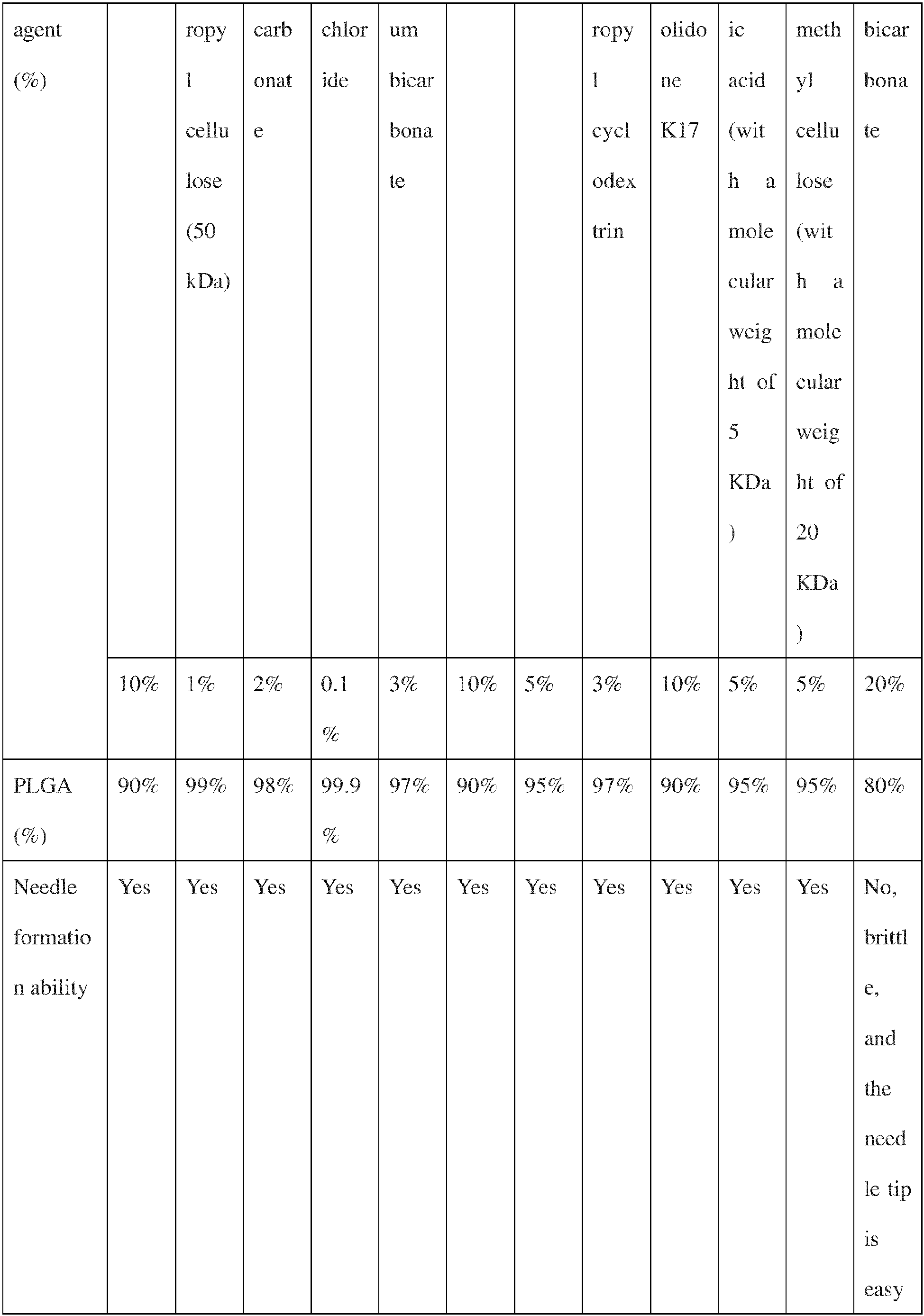

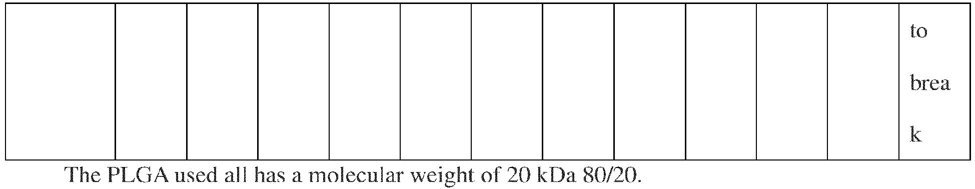

### Examples 18-28 Preparation of a small molecular drug-implanted type polylactic acid-based sustained-release microneedle patch

The preparation methods of Examples 18-28 are as shown in Example 1, and the specific parameters of each component in Examples 18-28 are shown in Table 2.

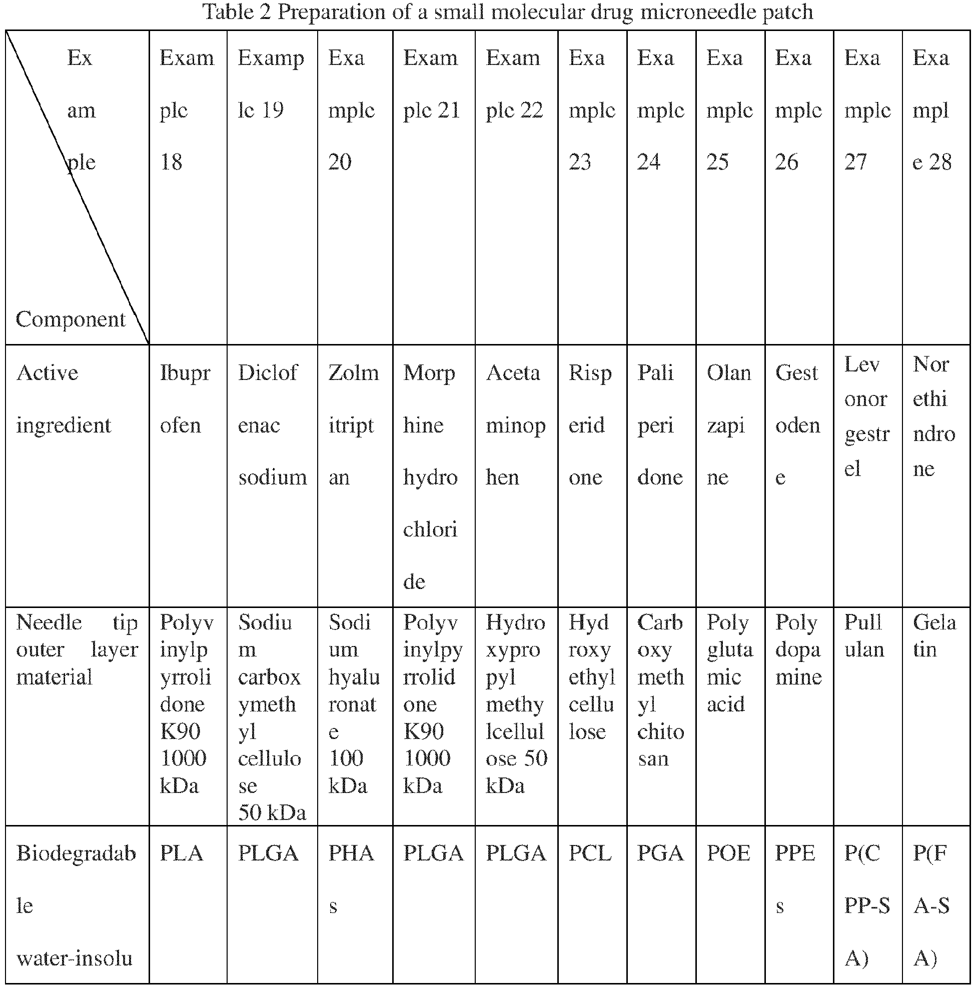

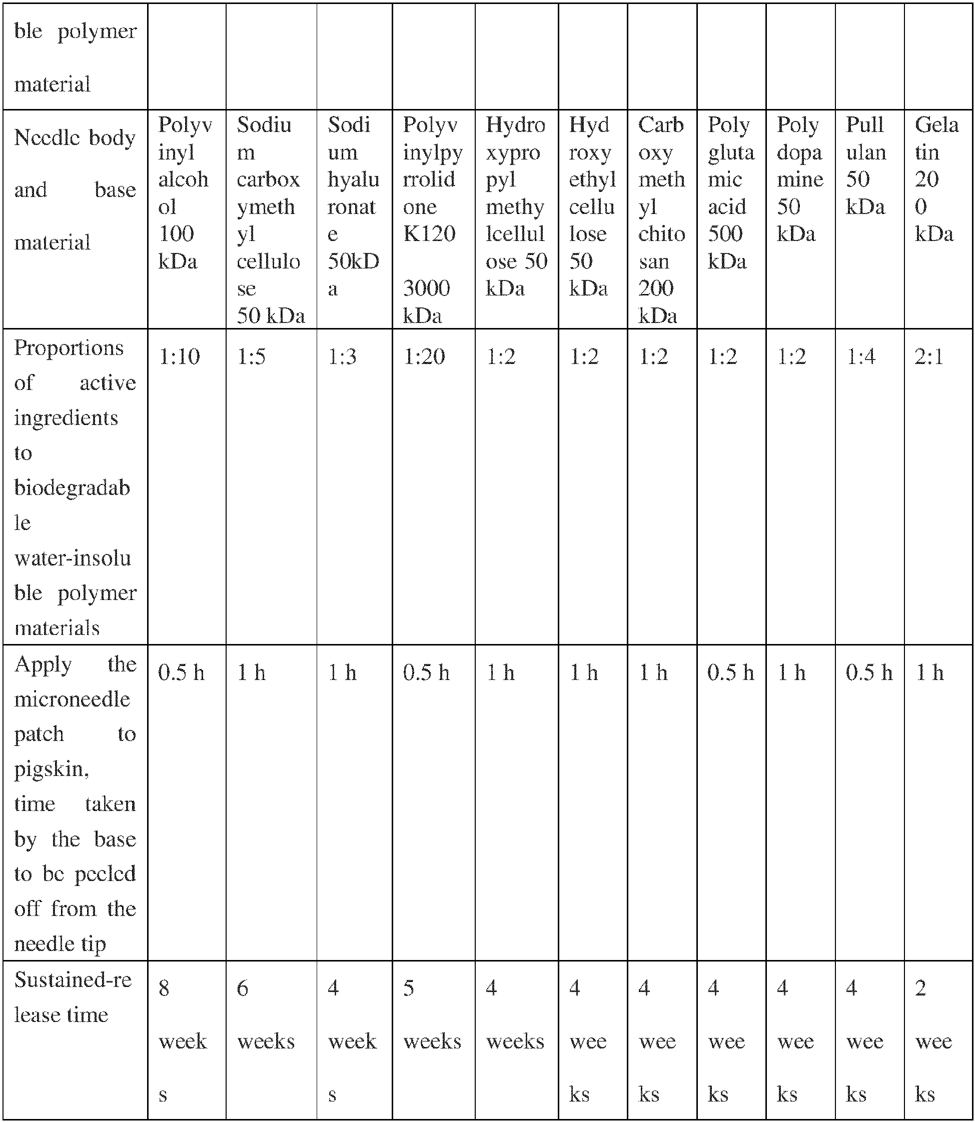

### Examples 29-35 Preparation of a macromolecular drug-implanted type polylactic acid-based sustained-release microneedle patch

The preparation methods of Examples 29-35 are as shown in Example 3, and the specific parameters of each component in Examples 26-35 are shown in Table 3.

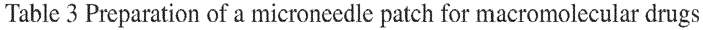

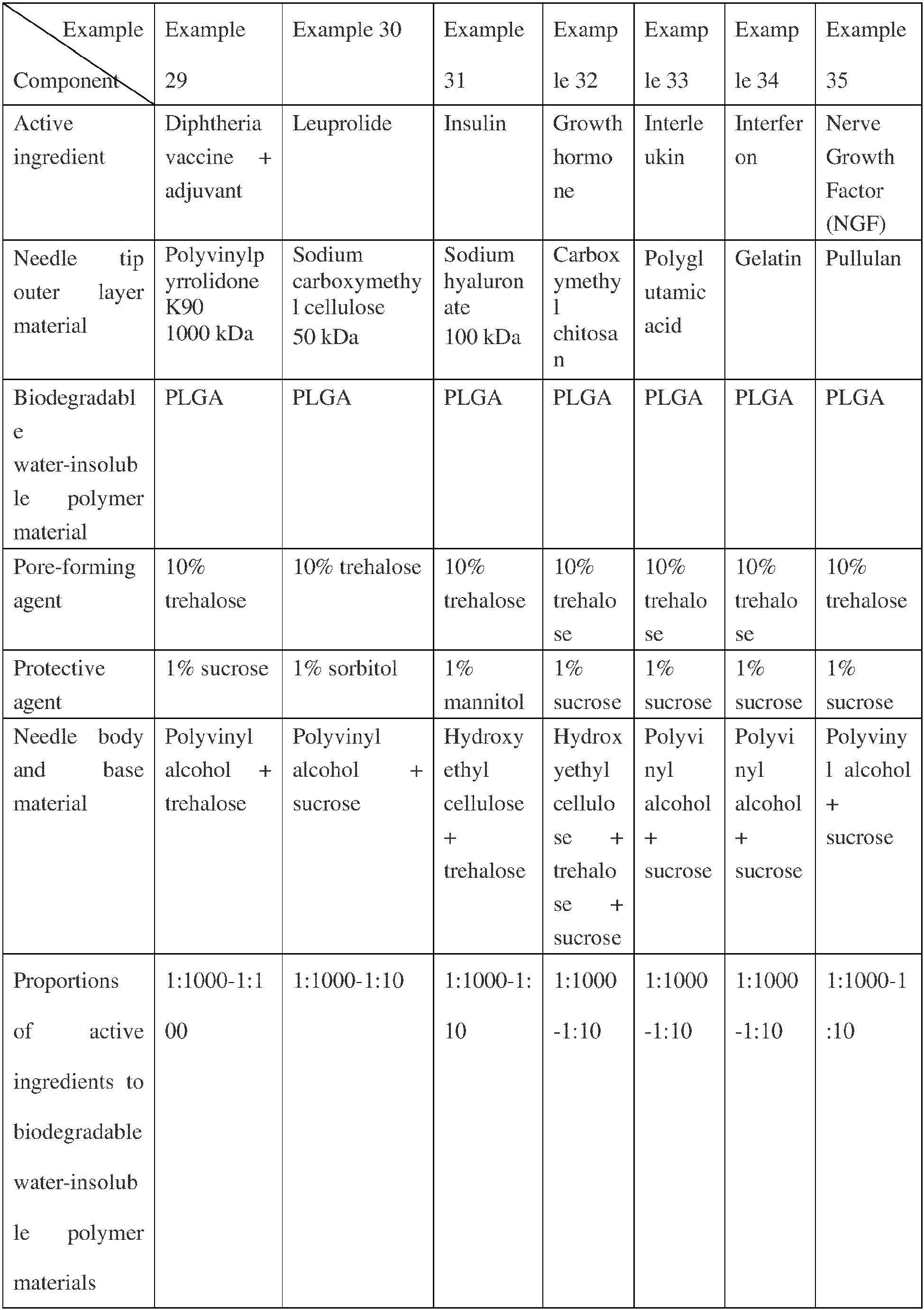

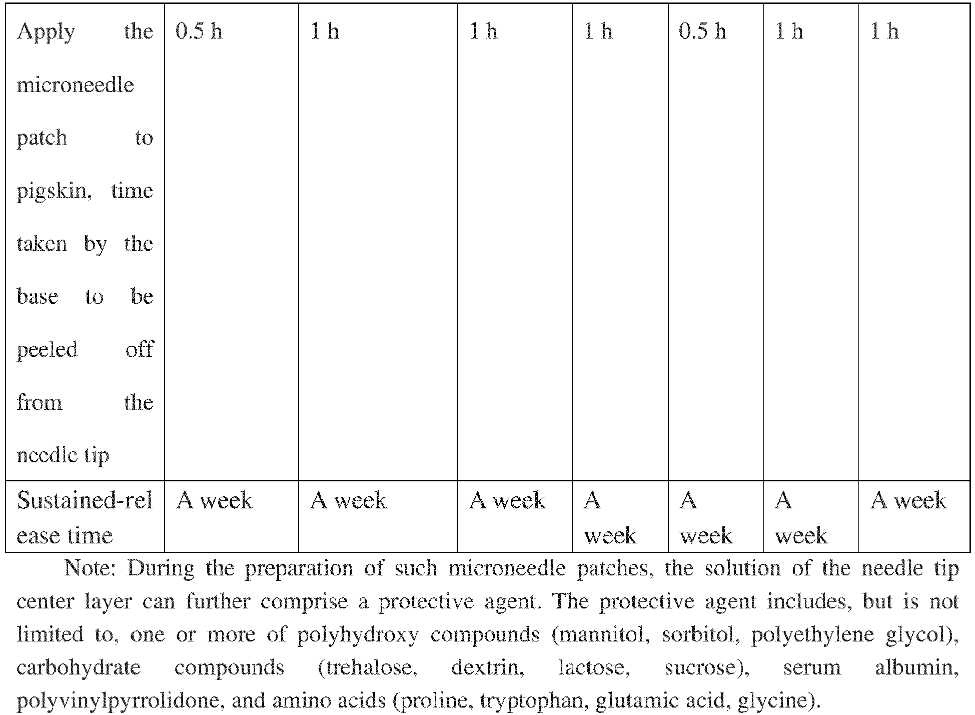

Examples 29-35 are also applicable to the preparation of other microneedle patches for macromolecular drugs or vaccines.

### Example 36 Preparation of implantable polylactic acid-based sustained-release microneedle patch for asiaticoside

1. 0.05g of sodium hyaluronate with a molecular weight of 50 kDa was taken and weighed, 4.95 ml of water was added, the resulting solution was placed and heated under 85 °C for 1 hour, to prepare a sodium hyaluronate aqueous solution with a mass fraction of 1% as a needle tip outer layer injection molding liquid. 3.5 g of polyvinyl alcohol with a molecular weight of 50 kDa and 0.35 g of trehalose were taken and weighed, 6.5 ml of water was added, the resulting solution was placed and heated under 85 °C for 2 hours, a polyvinyl alcohol-trehalose aqueous solution with a mass fraction of 35% was prepared as a needle body and base injection molding liquid.
2. 0.3g of PLGA (75/25) with a molecular weight of 20 kDa was taken and weighed, and 0.7 ml of N-**methyl**pyrrolidone was added, to prepare a 30% PLGA solution; 20 mg of asiaticoside was dispersed in the above-mentioned 30% PLGA solution, and the resulting solution was used as the the needle tip center layer injection molding liquid.
3. 10 µl of the above-mentioned needle tip outer layer injection molding liquid was added into a mold, the needle tip outer layer injection molding liquid was forced into pinholes of the microneedle mold by means of vacuuming, the excess solution on the mold was removed, and the mold was placed under 25 °C and 20% humidity to dry for 10 minutes; the above-mentioned PLGA solution containing asiaticoside was added into the above-mentioned microneedle mold, the needle tip center layer injection molding liquid was forced into the pinholes of the above-mentioned microneedle mold by means of vacuuming, the excess solution on the mold was removed, and the mold was heated under 60 °C for 1 hour; 150 µl of the above-mentioned needle body and base injection molding liquid was added to the above-mentioned cooled microneedle mold and vacuumed to avoid the formation of air bubbles; the above-mentioned microneedle mold was placed under 25 °C and 10% humidity to dry for 6 hours.
4. A pressure-sensitive adhesive backing was pasted on a backside of the base of the dried microneedle, and the microneedle patch was demoulded.

The prepared microneedle patch is as shown in FIG. 1. There are 49 needles in per square centimeter of the microneedle patch, and the height of the needle tip and the needle body is 1.5 mm.

If the microneedle patch is applied to pigskin, the base can be completely peeled off within 50 minutes, and the microneedle patch can be sustained-released for 15 days.

### Examples 37-42 Preparation of an implantable polylactic acid-based sustained-release microneedle patch containing cosmetic ingredients

The preparation methods of Examples 37-42 are as shown in Example 36, and the specific parameters of each component are shown in Table 6.

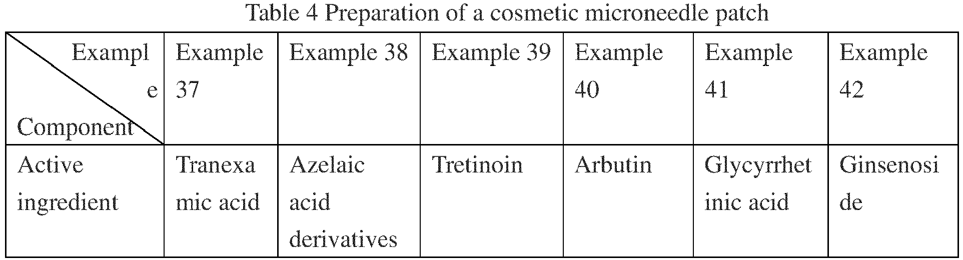

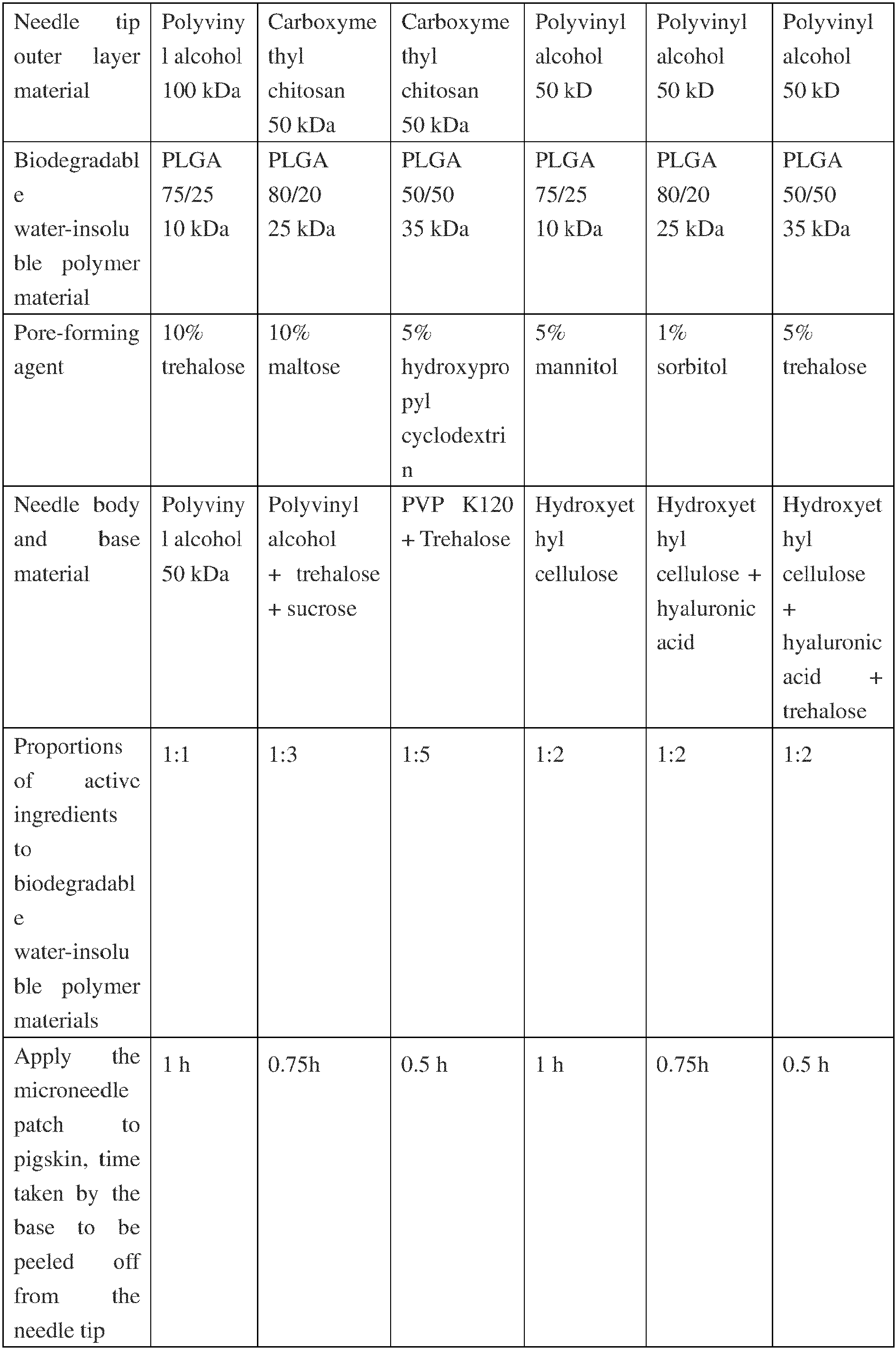

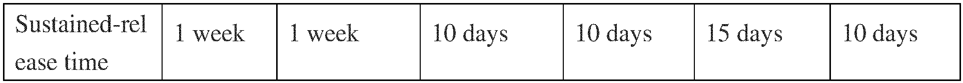

### Example 43 Skin puncturing experiments of a polylactic acid-based implantable sustained-release microneedle patch

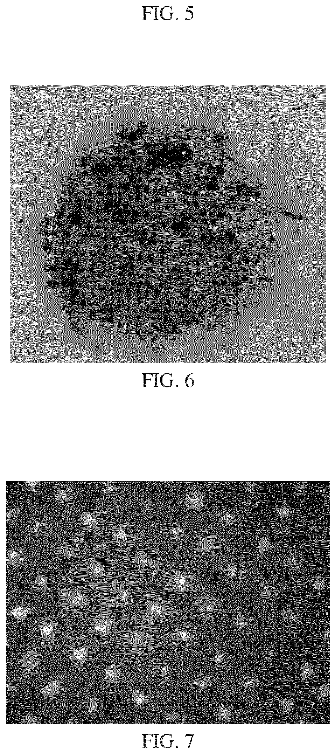

The microneedle patch prepared in Examples 1-42 (except Example 17) was applied to fresh pigskin, pressed with fingers for 1 min, and dyed with 1% concentration of trypan blue for 20 min, the excess trypan blue was wiped off with a cotton swab, and then whether there were pinholes on the skin was observed. As shown in FIG. 6 (taking Example 1 as an example), a photograph of trypan blue-dyed pigskin is shown. Microneedle pinholes can be clearly seen. The effects of other examples are the same as those in Example 1.

### Example 44 Intradermal implantation of a polylactic acid-based implantable sustained-release microneedle patch

The microneedle patch prepared in Examples 1-42 (except Example 17) was applied to fresh pigskin, pressed with fingers for 1 min, and the microneedle patch was kept on the skin for 1 hour, then an intradermal implantation situation of the needle tip was observed under a fluorescence microscope. FIG. 7 (taking Example 2 as an example) shows a photograph of the needle tip of the microneedle implanted in the skin under a fluorescence microscope. Array implantation of the fluorescent dye can be clearly seen. The effects of the microneedle patch in other examples are the same as those in Example 2.

It will be obvious that the above-mentioned examples of the present invention are merely examples to clearly illustrate the present invention, and are not intended to limit the embodiments of the present invention. For those of ordinary skill in the art, other different forms of variations or changes may also be made on the basis of the above description. It is not possible to list all the embodiments here. Any obvious variations or changes derived from the technical solutions of the present invention are still within the protection scope of the present invention.

## Claims

1. An implantable sustained-release microneedle, comprising a needle tip, a needle body, and a base; the needle tip comprising a needle tip center layer and a needle tip outer layer; the needle tip center layer being formed of a matrix comprising a biodegradable water-insoluble polymer material; and the needle tip outer layer, the needle body, and the base being formed of a matrix comprising a water-soluble polymer material, wherein the water-soluble polymer material of the needle tip outer layer is connected with the water-soluble polymer material of the needle body.

2. The implantable sustained-release microneedle according to claim 1, wherein the needle tip center layer comprises at least one active ingredient; preferably, a mass ratio of the biodegradable water-insoluble polymer material to the active ingredient is 0.5:1-1000:1.

3. The implantable sustained-release microneedle according to claim 1, wherein the biodegradable water-insoluble polymer material is one or more of polylactic acid, poly-L-lactide, poly-DL-lactide, poly(lactic-co-glycolic acid), polyglycolic acid, and polycaprolactone.

4. The implantable sustained-release microneedle according to claim 1, wherein a height of the needle tip center layer is less than or equal to two-thirds of a height of the needle tip and the needle body.

5. The implantable sustained-release microneedle according to claim 1, wherein the needle tip center layer further comprises a pore-forming agent; wherein the pore-forming agent is one or more of sodium chloride, sodium carbonate, sodium bicarbonate, ammonium bicarbonate, trehalose, maltose, cyclodextrin and its derivatives, polyvinylpyrrolidone, a low-molecular weight hyaluronic acid and its sodium salt, and low-molecular weight cellulose derivatives; preferably, the pore-forming agent accounts for 0.1%-10% of total mass of the needle tip center layer; more preferably, the needle tip center layer further comprises a protective agent; wherein the protective agent is one or more of a polyhydroxy compound, a carbohydrate compound, serum albumin, polyvinylpyrrolidone, chondroitin sulfate, and amino acids; more preferably, the protective agent accounts for 0.1%-10% of the total mass of the needle tip center layer.

6. The implantable sustained-release microneedle according to claim 1, wherein the water-soluble polymer material is one or more of carboxymethyl cellulose, hydroxypropyl methyl cellulose, hydroxyethyl cellulose, polyvinyl alcohol and its derivatives, polyvinylpyrrolidone and its derivatives, sodium hyaluronate and its derivatives, chondroitin sulfate, chitosan derivatives, polyacrylamide derivatives, polyglutamic acid, polydopamine, pullulan, gelatin, collagen, and silk protein; preferably, a molecular weight of the water-soluble polymer material is 20-2000 kDa.

7. The implantable sustained-release microneedle according to claim 1, wherein the needle tip outer layer, the needle body, and the base further comprise low-molecular weight carbohydrates and polyol compounds; preferably, the low-molecular weight carbohydrates and the polyol compounds are one or more of trehalose, sucrose, maltose, sorbitol, mannitol, xylitol, and glycerol.

8. A preparation method for the implantable sustained-release microneedle according to any one of claims 1-7, comprising the following steps:
mixing a biodegradable water-insoluble polymer material with a part of an organic solvent, adding or not adding a pore-forming agent, adding or not adding a protective agent, to prepare a needle tip center layer matrix material solution; mixing an active ingredient with the remaining organic solvent to prepare a drug solution; mixing the drug solution with the needle tip center layer matrix material solution to obtain a needle tip center layer injection molding liquid;
alternatively,
mixing the biodegradable water-insoluble polymer material with the organic solvent, adding or not adding the pore-forming agent, adding or not adding the protective agent, adding the active ingredient, and mixing uniformly to obtain the needle tip center layer injection molding liquid;
mixing the water-soluble polymer material with water, adding or not adding low-molecular weight carbohydrates or polyol compounds, and mixing uniformly to obtain a needle tip outer layer injection molding liquid; mixing the water-soluble polymer material with water, adding or not adding the low-molecular weight carbohydrates or the polyol compounds, and mixing uniformly to obtain a needle body and base injection molding liquid;
injecting the needle tip outer layer injection molding liquid into a microneedle mold, and drying to prepare a needle tip outer layer; injecting the needle tip center layer injection molding liquid into the microneedle mold, heating to remove the organic solvent, and cooling; injecting the needle body and base injection molding liquid into the microneedle mold to prepare a needle body and a base, and drying to obtain the implantable sustained-release microneedle.

9. An implantable sustained-release microneedle patch, comprising the implantable sustained-release microneedle according to any one of claims 1-7 and a backing.

10. The implantable sustained-release microneedle patch according to claim 9, wherein the microneedle patch is applied in fields of disease treatment and prevention, health care, and cosmetology.

## Patentansprüche

1. Implantierbare Mikronadel für anhaltende Freisetzung, umfassend eine Nadelspitze, einen Nadelkörper und eine Basis; wobei die Nadelspitze eine Nadelspitzen-Mittelschicht und eine Nadelspitzen-Außenschicht umfasst; wobei die Nadelspitzen-Mittelschicht aus einer Matrix gebildet ist, die ein biologisch abbaubares, wasserunlösliches Polymermaterial umfasst; und wobei die Nadelspitzen-Außenschicht, der Nadelkörper und die Basis aus einer Matrix gebildet sind, die ein wasserlösliches Polymermaterial umfasst, wobei das wasserlösliche Polymermaterial der Nadelspitzen-Außenschicht mit dem wasserlöslichen Polymermaterial des Nadelkörpers verbunden ist.

2. Implantierbare Mikronadel für anhaltende Freisetzung nach Anspruch 1, wobei die Nadelspitzen-Mittelschicht mindestens einen Wirkstoff umfasst; wobei ein Massenverhältnis des biologisch abbaubaren, wasserunlöslichen Polymermaterials zum Wirkstoff vorzugsweise 0,5:1-1000:1 beträgt.

3. Implantierbare Mikronadel für anhaltende Freisetzung nach Anspruch 1, wobei das biologisch abbaubare, wasserunlösliche Polymermaterial eines oder mehrere der folgenden ist: Polymilchsäure, Poly-L-Lactid, Poly-DL-Lactid, Poly(milch-co-glycolsäure), Polyglycolsäure und Polycaprolacton.

4. Implantierbare Mikronadel für anhaltende Freisetzung nach Anspruch 1, wobei die Höhe der Nadelspitzen-Mittelschicht weniger als oder gleich zwei Drittel der Höhe der Nadelspitze und des Nadelkörpers beträgt.

5. Implantierbare Mikronadel für anhaltende Freisetzung nach Anspruch 1, wobei die Nadelspitzen-Mittelschicht ferner ein porenbildendes Agens umfasst; wobei das porenbildende Agens eines oder mehrere der folgenden ist: Natriumchlorid, Natriumcarbonat, Natriumbicarbonat, Ammoniumbicarbonat, Trehalose, Maltose, Cyclodextrin und seine Derivate, Polyvinylpyrrolidon, eine Hyaluronsäure mit niedrigem Molekulargewicht und ihr Natriumsalz und Cellulosederivate mit niedrigem Molekulargewicht; wobei das porenbildende Agens bevorzugt 0,1%-10% zur Gesamtmasse der Nadelspitzen-Mittelschicht beiträgt; wobei die Nadelspitzen-Mittelschicht bevorzugter ferner ein Schutzmittel umfasst; wobei das Schutzmittel eines oder mehrere der folgenden ist: eine Polyhydroxyverbindung, eine Kohlenhydratverbindung, Serumalbumin, Polyvinylpyrrolidon, Chondroitinsulfat und Aminosäuren; wobei das Schutzmittel bevorzugter 0,1%-10% zur Gesamtmasse der Nadelspitzen-Mittelschicht beiträgt.

6. Implantierbare Mikronadel für anhaltende Freisetzung nach Anspruch 1, wobei das wasserlösliche Polymermaterial eines oder mehrere der folgenden ist: Carboxymethylcellulose, Hydroxypropylmethylcellulose, Hydroxyethylcellulose, Polyvinylalkohol und seine Derivate, Polyvinylpyrrolidon und seine Derivate, Natriumhyaluronat und seine Derivate, Chondroitinsulfat, Chitosanderivate, Polyacrylamidderivate, Polyglutaminsäure, Polydopamin, Pullulan, Gelatine, Kollagen und Seidenprotein; wobei ein Molekulargewicht des wasserlöslichen Polymermaterials bevorzugt 20-2000 kDa beträgt.

7. Implantierbare Mikronadel für anhaltende Freisetzung nach Anspruch 1, wobei die Nadelspitzen-Außenschicht, der Nadelkörper und die Basis weiterhin Kohlenhydrate mit niedrigem Molekulargewicht und Polyolverbindungen umfassen; wobei die Kohlenhydrate mit niedrigem Molekulargewicht und die Polyolverbindungen vorzugsweise eine oder mehrere der folgenden ist: Trehalose, Saccharose, Maltose, Sorbit, Mannit, Xylit und Glycerin.

8. Verfahren zur Herstellung der implantierbaren Mikronadel für anhaltende Freisetzung nach einem der Ansprüche 1-7, umfassend die folgenden Schritte:
Mischen eines biologisch abbaubaren, wasserunlöslichen Polymermaterials mit einem Teil eines organischen Lösungsmittels, Hinzufügen oder Nicht-Zufügen eines porenbildenden Agens, Hinzufügen oder Nicht-Zufügen eines Schutzmittels, um eine Nadelspitzen-Mittelschicht-Matrixmateriallösung herzustellen; Mischen eines Wirkstoffs mit dem verbleibenden organischen Lösungsmittel, um eine Arzneistofflösung herzustellen; Mischen der Arzneistofflösung mit der Nadelspitzen-Mittelschicht-Matrixmateriallösung, um eine Nadelspitzen-Mittelschicht-Spritzgießflüssigkeit zu erhalten;
alternativ,
Mischen des biologisch abbaubaren, wasserunlöslichen Polymermaterials mit dem organischen Lösungsmittel, Hinzufügen oder Nicht-Zufügen des porenbildenden Agens, Hinzufügen oder Nicht-Zufügen des Schutzmittels, Hinzufügen des Wirkstoffs und gleichmäßiges Mischen, um die Nadelspitzen-Mittelschicht-Spritzgießflüssigkeit zu erhalten;
Mischen des wasserlöslichen Polymermaterials mit Wasser, Zugeben oder Nicht-Zugeben von Kohlenhydraten mit niedrigem Molekulargewicht oder Polyolverbindungen und gleichmäßiges Mischen, um eine Nadelspitzen-Außenschicht-Spritzgießflüssigkeit zu erhalten; Mischen des wasserlöslichen Polymermaterials mit Wasser, Zugeben oder Nicht-Zugeben der Kohlenhydrate mit niedrigem Molekulargewicht oder der Polyolverbindungen und gleichmäßiges Mischen, um eine Nadelkörper- und Basis-Spritzgießflüssigkeit zu erhalten;
Einspritzen der Nadelspitzen-Außenschicht-Spritzgießflüssigkeit in eine Mikronadelgussform und Trocknen, um eine Nadelspitzen-Außenschicht herzustellen; Einspritzen der Nadelspitzen-Mittelschicht-Spritzgießflüssigkeit in die Mikronadelgussform, Erhitzen, um das organische Lösungsmittel zu entfernen, und Kühlen; Einspritzen der Nadelkörper- und Basis-Spritzgießflüssigkeit in die Mikronadelgussform, um einen Nadelkörper und eine Basis herzustellen, und Trocknen, um die implantierbare Mikronadel für anhaltende Freisetzung zu erhalten.

9. Pflaster mit implantierbarer Mikronadel für anhaltende Freisetzung, umfassend die implantierbare Mikronadel für anhaltende Freisetzung nach einem der Ansprüche 1-7 und eine Unterschicht.

10. Pflaster mit implantierbarer Mikronadel für anhaltende Freisetzung nach Anspruch 9, wobei das Mikronadelpflaster in Bereichen der Krankheitsbehandlung und -vorbeugung, Gesundheitsfürsorge und Kosmetologie eingesetzt wird.

## Revendications

1. Micro-aiguille implantable à libération prolongée, comprenant une pointe d'aiguille, un corps d'aiguille et une base ; la pointe d'aiguille comprenant une couche centrale de pointe d'aiguille et une couche extérieure de pointe d'aiguille ; la couche centrale de pointe d'aiguille étant formée d'une matrice comprenant un matériau polymère biodégradable insoluble dans l'eau ; et la couche extérieure de pointe d'aiguille, le corps d'aiguille et la base étant formés d'une matrice comprenant un matériau polymère soluble dans l'eau, dans laquelle le matériau polymère soluble dans l'eau de la couche extérieure de pointe d'aiguille est relié au matériau polymère soluble dans l'eau du corps d'aiguille.

2. Micro-aiguille implantable à libération prolongée selon la revendication 1, dans laquelle la couche centrale de pointe d'aiguille comprend au moins un ingrédient actif ; de préférence, le rapport en masse entre le matériau polymère biodégradable insoluble dans l'eau et l'ingrédient actif est de 0,5:1 à 1 000:1.

3. Micro-aiguille implantable à libération prolongée selon la revendication 1, dans laquelle le matériau polymère biodégradable insoluble dans l'eau est un ou plusieurs parmi l'acide polylactique, le poly-L-lactide, le poly-DL-lactide, le poly(acide lactique-co-glycolique), l'acide polyglycolique et la polycaprolactone.

4. Micro-aiguille implantable à libération prolongée selon la revendication 1, dans laquelle une hauteur de la couche centrale de pointe d'aiguille est inférieure ou égale aux deux-tiers d'une hauteur de la pointe d'aiguille et du corps d'aiguille.

5. Micro-aiguille implantable à libération prolongée selon la revendication 1, dans laquelle la couche centrale de pointe d'aiguille comprend en outre un agent de formation de pores ; dans laquelle l'agent de formation de pores est un ou plusieurs parmi le chlorure de sodium, le carbonate de sodium, le bicarbonate de sodium, le bicarbonate d'ammonium, le tréhalose, le maltose, la cyclodextrine et ses dérivés, la polyvinylpyrrolidone, un acide hyaluronique de faible poids moléculaire et son sel de sodium, et les dérivés de cellulose de faible poids moléculaire ; de préférence, l'agent de formation de pores représente de 0,1 % à 10 % de la masse totale de la couche centrale de pointe d'aiguille ; de manière davantage préférée, la couche centrale de pointe d'aiguille comprend en outre un agent protecteur ; dans laquelle l'agent protecteur est un ou plusieurs parmi un composé polyhydroxy, un composé hydrate de carbone, la sérum-albumine, la polyvinylpyrrolidone, le sulfate de chondroïtine et des acides aminés ; de manière davantage préférée, l'agent protecteur représente de 0,1 % à 10 % de la masse totale de la couche centrale de pointe d'aiguille.

6. Micro-aiguille implantable à libération prolongée selon la revendication 1, dans laquelle le matériau polymère soluble dans l'eau est un ou plusieurs parmi la carboxyméthylcellulose, l'hydroxypropylméthylcellulose, l'hydroxyéthylcellulose, le poly(alcool de vinyle) et ses dérivés, la polyvinylpyrrolidone et ses dérivés, le hyaluronate de sodium et ses dérivés, le sulfate de chondroïtine, les dérivés de chitosane, les dérivés de polyacrylamide, l'acide polyglutamique, la polydopamine, le pullulane, la gélatine, le collagène et la protéine de soie ; de préférence, un poids moléculaire du matériau polymère soluble dans l'eau est de 20 à 2 000 kDa.

7. Micro-aiguille implantable à libération prolongée selon la revendication 1, dans laquelle la couche extérieure de pointe d'aiguille, le corps d'aiguille et la base comprennent en outre des hydrates de carbone de faible poids moléculaire et des composés polyol ; de préférence, les hydrates de carbone de faible poids moléculaire et les composés polyol sont un ou plusieurs parmi le tréhalose, le saccharose, le maltose, le sorbitol, le mannitol, le xylitol et le glycérol.

8. Procédé de préparation de la micro-aiguille implantable à libération prolongée selon l'une quelconque des revendications 1 à 7, comprenant les étapes suivantes :
mélange d'un matériau polymère biodégradable insoluble dans l'eau avec une partie d'un solvant organique, ajout ou non-ajout d'un agent de formation de pores, ajout ou non-ajout d'un agent protecteur, pour préparer une solution de matériau de matrice de couche centrale de pointe d'aiguille ; mélange d'un ingrédient actif avec le solvant organique restant pour préparer une solution de médicament ; mélange de la solution de médicament avec la solution de matériau de matrice de couche centrale de pointe d'aiguille pour obtenir un liquide de moulage par injection de couche centrale de pointe d'aiguille ;
en variante,
mélange du matériau polymère biodégradable insoluble dans l'eau avec le solvant organique, ajout ou non-ajout de l'agent de formation de pores, ajout ou non-ajout de l'agent protecteur, ajout de l'ingrédient actif et mélange uniforme pour obtenir le liquide de moulage par injection de couche centrale de pointe d'aiguille ;
mélange du matériau polymère soluble dans l'eau avec de l'eau, ajout ou non-ajout d'hydrates de carbone de faible poids moléculaire ou de composés polyol, et mélange uniforme pour obtenir un liquide de moulage par injection de couche extérieure de pointe d'aiguille ; mélange du matériau polymère soluble dans l'eau avec de l'eau, ajout ou non-ajout des hydrates de carbone de faible poids moléculaire ou des composés polyol, et mélange uniforme pour obtenir un liquide de moulage par injection de corps d'aiguille et de base ;
injection du liquide de moulage par injection de couche extérieure de pointe d'aiguille dans un moule à micro-aiguille, et séchage pour préparer une couche extérieure de pointe d'aiguille ; injection du liquide de moulage par injection de couche centrale de pointe d'aiguille dans le moule à micro-aiguille, chauffage pour retirer le solvant organique et refroidissement ; injection du liquide de moulage par injection de corps d'aiguille et de base dans le moule à micro-aiguille pour préparer un corps d'aiguille et une base, et séchage pour obtenir la micro-aiguille implantable à libération prolongée.

9. Patch à micro-aiguille implantable à libération prolongée, comprenant la micro-aiguille implantable à libération prolongée selon l'une quelconque des revendications 1 à 7 et un support.

10. Patch à micro-aiguille implantable à libération prolongée selon la revendication 9, dans lequel le patch à micro-aiguille est appliqué dans les domaines de la prévention et du traitement des maladies, des soins de santé et de la cosmétologie.
